(19)

Europäisches
Patentamt
European
Patent Office
Office européen
des brevets

(11) **EP 3 406 734 A1**

(12) **EUROPEAN PATENT APPLICATION**
published in accordance with Art. 153(4) EPC

(43) Date of publication:
**28.11.2018  Bulletin 2018/48**

(21) Application number: **17741357.2**

(22) Date of filing: **16.01.2017**

(51) Int Cl.:
*C12Q 1/68* $^{(2018.01)}$      *C12M 1/00* $^{(2006.01)}$
*C12N 15/09* $^{(2006.01)}$     *G01N 21/78* $^{(2006.01)}$
*G01N 35/00* $^{(2006.01)}$

(86) International application number:
**PCT/JP2017/001280**

(87) International publication number:
**WO 2017/126479 (27.07.2017 Gazette 2017/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **22.01.2016  JP 2016011018**

(71) Applicants:
• **ARKRAY, Inc.**
  **Minami-ku**
  **Kyoto-shi**
  **Kyoto 601-8045 (JP)**
• **National University Corporation
  Kyoto Institute of Technology
  Sakyo-ku
  Kyoto-shi, Kyoto 606-8585 (JP)**

(72) Inventors:
• **MURAKAMI, Akira**
  **Kyoto-shi**
  **Kyoto 606-8585 (JP)**
• **KOBORI, Akio**
  **Kyoto-shi**
  **Kyoto 606-8585 (JP)**
• **YAMAYOSHI, Asako**
  **Kyoto-shi**
  **Kyoto 606-8585 (JP)**
• **NODA, Yuichiro**
  **Kyoto-shi**
  **Kyoto 602-0008 (JP)**

(74) Representative: **Dehns**
  **St. Brides House
  10 Salisbury Square
  London EC4Y 8JD (GB)**

(54) **TARGET ANALYSIS METHOD AND TARGET ANALYSIS CHIP**

(57)    A novel target analysis method and target analyzing chip are provided to directly detect a target, such as microRNA, without performing PCR. The target analysis method of the present embodiment includes: a process in which a target in a sample, a labeled probe that binds to the target, and a carrier-binding probe that binds to the target and to a carrier, contact each other, and the target, the labeled probe, and the carrier-binding probe react to bind to each other to form a first bound body; a process in which the first bound body and the carrier contact each other, and the first bound body and the carrier react to bind to each other to form a second bound body; a process to concentrate the carrier; and a process in which the target in the sample is analyzed by detecting a label of the labeled probe bound to the concentrated carrier.

**EP 3 406 734 A1**

**(Cont. next page)**

FIG.4A

EXAMPLE 1

COMPARATIVE
EXAMPLE 1A

COMPARATIVE
EXAMPLE 1B

COMPARATIVE
EXAMPLE 1C

COMPARATIVE
EXAMPLE 1D

FIG.4B

EXAMPLE 1

COMPARATIVE
EXAMPLE 1A

COMPARATIVE
EXAMPLE 1B

COMPARATIVE
EXAMPLE 1C

COMPARATIVE
EXAMPLE 1D

2

Sequence Listing

SEQUENCE LISTING

<110>  Arkray, Inc.
        National University Corporation Kyoto Institute of
        Technology

<120>  Method for analyzing a target and chip for analyzing a target

<130>  T15064-01

<160>  3

<170>  PatentIn version 3.5

<210>  1
<211>  30
<212>  RNA
<213>  Artificial Sequence

<220>
<223>  polynucelotide

<400>  1
agucaauagg gugugugaga gacuuaacug                                      30

<210>  2
<211>  14
<212>  RNA
<213>  Artificial Sequence

<220>
<223>  probe

<220>
<221>  misc_feature
<222>  (5)..(6)
<223>  n is pyrene labeled cytidine

<400>  2
acacnnuauu gacu                                                       14

<210>  3
<211>  20
<212>  DNA
<213>  Artificial Sequence

<220>
<223>  probe

<400>  3
cagccagtta agtctctcac                                                 20

**EP 3 406 734 A1**

**Description**

Technical Field

The present invention relates to a target analysis method and a target analyzing chip.

Background Art

There has recently been interest shown in associations between RNA, such as microRNA in blood, and diseases. Attempts are being made to utilize detection of such RNA in medical treatments, such as in the early detection of diseases.
As a microRNA quantitation method, a method has been reported in which, for example, target microRNA is specifically amplified for detection by performing a polymerase chain reaction (PCR) (Patent Document 1). However, temperature control devices, DNA reverse transcription, and so on are required due to the necessity of the PCR, and this makes operation complicated. Moreover, in such methods, a DNA is obtained from an RNA contained in a small amount of sample using reverse transcription, and then, in addition, the DNA is amplified by performing the PCR. However, in such reverse transcription PCR, the RNA in the sample is detected indirectly, with this leading to an issue that precision is insufficient for quantitative analysis due to not detecting the RNA directly.

Prior Art

Patent Documents

Patent Document 1: JP 5192229 B

SUMMARY OF INVENTION

Technical Problem

Thus, an object of the present disclosure is to provide a novel target analysis method and target analyzing chip to directly detect a target such as microRNA without the necessity of PCR.

Solution to Problem

In order to achieve the object of the present disclosure, a target analysis method of the present embodiment includes a first reaction process, a second reaction process, a concentration process, and an analysis process. The first reaction process is a process in which a target in a sample, a labeled probe that binds to the target, and a carrier-binding probe that binds to the target and to a carrier formed from a light-transmitting material contact each other in a liquid solvent, and the target, the labeled probe, and the carrier-binding probe react to bind to each other to form a first bound body. The second reaction process is a process in which the first bound body and the carrier contact each other, and the first bound body and the carrier react to bind to each other to form a second bound body having a specific gravity higher than that of the liquid solvent. The concentration process is a process in which the liquid solvent containing the second bound body is centrifuged to concentrate the second bound body. The analysis process is a process in which the target in the sample is analyzed by detecting a label of the labeled probe contained in the concentrated second bound body.
A target analyzing chip of the present embodiment includes a base plate provided with a reaction section, a detection section, and a flow path that communicates the reaction section with the detection section. The reaction section is a location where a target in a sample, a labeled probe that binds to the target, and a carrier-binding probe that binds to the target and to a carrier formed from a light-transmitting material, and the carrier react to bind to each other. The detection section is a location where a second bound body is concentrated, the second bound body being formed by a binding between a first bound body, in which the target, the labeled probe, and the carrier-binding probe are bound together, and the carrier via the carrier-binding probe. The flow path includes a hydrophobic internal wall as a movement control means to control movement of the second bound body from the reaction section to the detection section. When a centrifugal force (C) greater than a resistance force (R) arising due to the hydrophobic properties of the flow path is applied, in a liquid solvent, the second bound body having a specific gravity higher than that of the liquid solvent can be moved by the movement control means to the detection section through the flow path. Advantageous Effects
The present embodiment enables a target in a sample to be directly analyzed without using PCR.

3

BRIEF DESCRIPTION OF DRAWINGS

Figs. 1A to 1D are schematic diagrams illustrating an example of a target analyzing chip of the present embodiment, Fig. 1A is a perspective view, Fig. 1B is a plan view, and Fig. 1C is a cross-section taken along the direction I-I of Fig. 1A.

Fig. 2 is a cross-section illustrating an example of a centrifuge.

Fig. 3 is a schematic diagram illustrating an example of a state in which a target analyzing chip is placed on a centrifuge.

Figs. 4A and 4B are photographs taken when measuring avidin-modified beads on a glass bottom dish in Example 1 of the present embodiment.

Fig. 5 is a graph illustrating a spectrum of a fluorescent signal for the Example 1 of the present embodiment.

Fig. 6A is a graph illustrating average fluorescence intensities for Examples 2, and Fig. 6B is a graph illustrating absorbances for Examples 2.

Figs. 7A to 7C are plan views of the interior of analyzing chips employed in Examples 3.

Figs. 8A and 8B are graphs illustrating relative values of total amounts of fluorescence in the Examples 3.

DESCRIPTION OF EMBODIMENTS

A target analysis method of the present embodiment includes, as mentioned above: a first reaction process in which a target in a sample, a labeled probe that binds to the target, and a carrier-binding probe that binds to the target and to a carrier formed from a light-transmitting material contact each other in a liquid solvent, and the target, the labeled probe, and the carrier-binding probe react to bind to each other to form a first bound body; a second reaction process in which the first bound body and the carrier contact each other, and the first bound body and the carrier react to bind to each other to form a second bound body having a specific gravity higher than that of the liquid solvent; a concentration process in which the liquid solvent containing the second bound body is centrifuged to concentrate the second bound body; and an analysis process in which the target in the sample is analyzed by detecting a label of the labeled probe contained in the concentrated second bound body.

In the target analysis method of the present embodiment, for example, the carrier-binding probe includes a first binding substance, the carrier includes a second binding substance that binds to the first binding substance, and the carrier-binding probe binds to the carrier through binding between the first binding substance to the second binding substance.

The target of the target analysis method of the present embodiment is a target nucleic acid.

In the target analysis method of the present embodiment, the target nucleic acid is, for example, target RNA. The target RNA is, for example, microRNA.

In the target analysis method of the present embodiment, the labeled probe is, for example, a fluorescent labeled probe.

In the target analysis method of the present embodiment, the fluorescent labeled probe, for example, binds closer to a 5' end side of the target nucleic acid than the carrier-binding probe.

In the target analysis method of the present embodiment, the fluorescent labeled probe binds, for example, to contiguous bases from the 5' end of the target nucleic acid, and the carrier-binding probe binds to contiguous bases from a 3' end of the target nucleic acid.

In the target analysis method of the present embodiment, the fluorescent label of the fluorescent labeled probe is, for example, a substance that undergoes a signal alteration through a binding of the fluorescent labeled probe to the target nucleic acid.

In the target analysis method of the present embodiment, the fluorescent label is, for example, a substance that includes pyrene.

The target analysis method of the present embodiment further includes a process in which psoralen, which additionally modifies the labeled probe, and the target nucleic acid are caused to bind together by irradiation with ultraviolet light.

In the target analysis method of the present embodiment, fluorescence is detected by, for example, detecting a fluorescent signal at a wavelength from 450 nm to 510 nm.

In the target analysis method of the present embodiment, the diameter of the carrier is, for example, less than 100 $\mu$m, and is more preferably from 100 nm to 4 $\mu$m.

The target analysis method of the present embodiment, for example, employs a target analyzing chip including a base plate. The base plate is provided with a reaction section, a detection section, and a flow path that communicates the reaction section with the detection section. The reaction section is a location where the target, the labeled probe, the carrier-binding probe, and the carrier react to bind to each other. The detection section is a location where the second bound body is concentrated. The flow path includes a hydrophobic internal wall as a movement control means to control movement of the second bound body from the reaction section to the detection section . When a centrifugal force (C) greater than a resistance force (R) arising due to the hydrophobic properties of the flow path is applied, the second bound body can be moved by the movement control means to the detection section through the flow path. After a sample containing the target is introduced into the reaction section, the first reaction process is performed in the reaction section. The second reaction process is then also performed in the reaction section, the concentration process is performed en route from the reaction section to the detection section through the flow path, and the analysis process is performed in the detection section.

In the target analysis method of the present embodiment, the height of the detection section is, for example, from 1 $\mu$m to 500 $\mu$m.

In the target analysis method of the present embodiment, the carrier is, for example, configured by a bead. Moreover, the carrier is, for example, made from silica.

In the target analysis method of the present embodiment, the diameter of the bead is, for example, from 100 nm to 4 $\mu$m.

Target Analysis Method

The target analysis method of the present embodiment (also sometimes referred to as "analysis method" hereinafter) includes, as stated above: a process in which a target in a sample, a labeled probe that binds to the target, and a carrier-binding probe that binds to the target and to a carrier formed from a light-transmitting material contact each other in a liquid solvent, and the target, the labeled probe, and the carrier-binding probe react to bind to each other to form a first bound body (the first reaction process); a process in which the first bound body and the carrier contact each other, and the first bound body and the carrier react to bind to each other to form a second bound body having a specific gravity higher than that of the liquid solvent (the second reaction process); a process in which the liquid solvent containing the second bound body is centrifuged to concentrate the second bound body (the concentration process); and a process in which the target in the sample is analyzed by detecting a label of the labeled probe contained in the second bound body in a concentrated state (the analysis process). The analysis method of the present embodiment includes the first reaction process, the second reaction process, the concentration process, and the analysis process, and there are no particular limitations to other processes or conditions. The analysis method of the present embodiment is able, in the first reaction process and the second reaction process, to concentrate the first bound body, which is a combination of the target, the labeled probe, and the carrier-binding probe, on the carrier via the carrier-binding probe. The analysis method of the present embodiment is also able to concentrate the carrier in the concentration process. Thus, the analysis method of the present embodiment is, for example, able in the analysis process to analyze the carrier to which the first bound body containing the target is concentrated and bound (the second bound body) in a highly concentrated state. Accordingly, the analysis method of the present embodiment, for example, enables excellent sensitivity and precision to be implemented, and enables analysis to be performed even, for example, with only a very small amount of the sample provided. Moreover, the analysis method of the present embodiment, for example, enables the carrier to be analyzed in a highly concentrated state, thereby enabling a situation to be avoided in which a labeled probe not bound to the target affects the analysis. Therefore, the analysis method of the present embodiment reduces background noise, for example. Moreover, the analysis method of the present embodiment enables the binding reaction between the target, the labeled probe, and the carrier-binding probe to be performed in a dispersed state. Moreover, the analysis method of the present embodiment enables the first bound body and the carrier to be bound together via the carrier-binding probe in the first bound body obtained. Thus, due to there being a higher probability of the binding reaction between the labeled probe and the target dispersed in the reaction solution occurring in the first reaction process, the analysis method of the present embodiment, for example, enables the time of the binding reaction to be further shortened and furthermore the sensitivity to be further improved, compared to analysis methods in which the carrier with immobilized labeled probe is employed to analyze the target, or analysis methods in which a bound body in which the carrier-binding probe and the carrier are preliminarily bound is employed to analyze the target. the analysis method of the present embodiment may, for example, be a qualitative analysis in which the presence or absence of a target in the sample is analyzed, or may be a quantitative analysis in which the extent of a target in the sample is analyzed. The target analysis method of the present embodiment does not include PCR processing.

In a sample analyzed in the present embodiment, the sample is a liquid sample. The liquid sample is, for example, a biological specimen, and may, moreover, be a dilution or suspension of the specimen, or the like. Examples of the specimen include body fluids such as blood, urine, gastric juices, sputum, amniotic fluid, and peritoneal fluid, tissues of large intestines or lungs, or the like, and cells such as oral cells, germ cells, and cells, nail cells, hair cells, or the like. Examples of blood include whole blood, serum, blood plasma, a hemolyzed solution, or the like. The liquid sample may, for example, be pre-processed. Such pre-processing is not particularly limited, and examples include processing in which the target (such as nucleic acids of an RNA or a DNA nucleic acid) is released from cells contained in the specimen. In the analysis method of the present embodiment, the sample may, for example, be a sample containing a target, may be a sample that does not contain the target, or may be a sample in which it is not known whether a target is contained or not.

In the analysis method of the present embodiment, there are no particular limitations to the type of the target, and examples include target nucleic acids etc. Examples of the target nucleic acids include a target RNA, a target DNA, etc. The target RNA is not particularly limited, and examples include a microRNA, an RNA from viruses, a messenger RNA, etc. The target DNA is not particularly limited, and examples of the DNA include a genomic DNA, a fragment thereof, a cDNA, a free DNA in blood, and a DNA included in blood-circulating tumor cells.

The labeled probe is a probe modified by a labeling substance (referred to as a "label" hereinafter). Examples of the probe include an RNA and a DNA, and the probe is preferably an RNA. The RNA may be an unmodified RNA or a modified RNA, or may contain a combination thereof. Examples of the modified RNA include a 2' -O-methyl RNA. Moreover, the DNA may be a modified DNA, an unmodified DNA, or may contain a combination thereof. The probe may include synthetic nucleic acid monomers. Examples of synthetic nucleic acid monomers include peptide nucleic acids (PNA), locked nucleic acids (LNA), 2'-O, 4'-C-ethylenebridged nucleic acids (ENA), and the like. The method of modifying the probe with the labeling substance is not particularly limited, and any known methods may be employed therefor. The sequence of the labeled probe can, for example, be designed appropriately based on the target to be analyzed. In cases in which the target is a target nucleic acid, examples of the sequence of the labeled probe include sequences partially or fully complementary to that of the target nucleic acid. A labeling position of the labeled probe is not particularly limited, and may be, for example, a base at the 3' terminal of the labeled probe, a base at the 5' terminal of the labeled probe, or another base.

The labeling substance is not particularly limited, and examples include fluorescent labeling substances, radioactive labeling substance, and the like. The fluorescent labeling substance is not particularly limited, and examples include fluorescent substances such as fluorophores. Examples of the fluorescent labeling substance include fluorescein, phosphorescence, rhodamine, and derivatives of polymethyl pigments. Examples of commercial fluorescent labeling substances include Pacific Blue (registered trademark, manufactured by Molecular Probes), BODIPY FL (registered trademark, manufactured by Molecular Probes), FlorePrime (trade name, manufactured by Amersham Pharmacia Biotech Ltd.), Fluoredite (trade name, manufactured by Millipore Corporation), FAM (registered trademark, manufactured by ABI Corporation), Cy3 and Cy5 (trade names, manufactured by Amersham Pharmacia Biotech Ltd.), and TAMRA (registered trademark, manufactured by Molecular Probes).

In cases in which the labeled probe is a fluorescent labeled probe labeled with the fluorescent labeling substance, the fluorescent labeling substance of the fluorescent labeled probe is, for example, preferably a substance that undergoes signal alteration through a binding of the fluorescent labeled probe to the target (a target nucleic acid, for example). The signal is, for example, a fluorescent signal. Such a fluorescent labeling substance as described above, for example, eliminates the need to separate the fluorescent labeled probe bound to the target from the fluorescent labeled probe not bound to the target, thereby enabling precision of analysis to be improved and analysis to be further simplified.

Specific examples of the fluorescent labeling substance include substances containing pyrene (also referred to as "pyrene-containing substances" hereinafter). The fluorescent labeling substance may, for example, contain pyrene, or may be pyrene itself. In cases in which the fluorescent labeling substance is a pyrene-containing substance, the description in JP 4238381 B, for example, may be invoked for a design of the fluorescent labeled probe. It is preferable for the pyrene-containing substance, for example, to modify uridine residue and/or cytidine residue, and, specifically, it is preferable for the pyrene-containing substance to bind covalently to the 2'-hydroxyl group of the uridine residue and/or to the 2'-hydroxyl group of the cytidine residue. The fluorescent labeled probe is, for example, preferably an RNA containing uridine to which the pyrene-containing substance covalently bonds, or a DNA containing uridine and/or cytidine to which the pyrene-containing substance covalently bonds. The fluorescent labeled probe modified by the pyrene-containing substance, for example, binds to the target nucleic acid (a target RNA, for example), and emits fluorescence by forming a double-stranded structure therewith. This enables the target nucleic acid to be analyzed by detecting a signal of the fluorescence. The fluorescence may, for example, be generated by excitation by ultraviolet light irradiation. The wavelength of the irradiation light (excitation light) is set, for example, from 325 nm to 350 nm (preferably 340 nm), and the fluorescence detection wavelength is set, for example, from 450 nm to 510 nm (preferably 480 nm).

It is preferable for the fluorescent labeled probe to be further modified by psoralen, for example. In cases in which the fluorescent labeled probe contains psoralen, for example, when ultraviolet light is irradiated onto the double-strand formed by the fluorescent labeled probe and the target nucleic acid, the psoralen of the fluorescent labeled probe covalently bonds to the uracil residue of the target nucleic acid in the double-stranded structure. This enables the double-strand bonding between the target nucleic acid and fluorescent labeled probe to be further stabilized. This accordingly, for example, make it possible to sufficiently prevent the target nucleic acid to separate from the fluorescent labeled probe, and also enables sensitivity to be further improved, even while the various processes described below are being implemented with the analyzing chip.

The length of the labeled probe is not particularly limited, and may be determined, for example, appropriately according to the type of target. In cases in which the target is the target nucleic acid, the length of the labeled probe may be set appropriately according to the length of the target nucleic acid, for example. In cases in which in the target nucleic acid is a microRNA, the length of the labeled probe is, for example, 7 to 15 bases long.

In the analysis method of the present embodiment, the carrier-binding probe is a probe modified by a label capable of binding to the carrier (a binding label). Examples of the probe include an RNA and a DNA. The RNA may, for example, be an unmodified RNA, may be a modified RNA, or may contain a combination thereof. Examples of the modified RNA include 2'-O-methyl RNA and the like. The DNA may, for example, be a modified DNA, may be an unmodified DNA, or may contain a combination thereof. The carrier-binding probe may, for example, contain synthetic nucleic acid monomers. The method for modifying the carrier-binding probe with the binding label is not particularly limited, and any known methods may be employed therefor according to the type of the binding label. The sequence of the carrier-binding probe may be designed appropriately based on the target to be analyzed, for example. In cases in which the target is the target nucleic acid, examples of the sequence of the carrier-binding probe include sequences partially or fully complementary to that of the target nucleic acid. The number of the binding labels in the carrier-binding probe is not particularly limited, and may be set appropriately according to the type of the binding label.

The binding label is, for example, a label that binds specifically to the carrier. Such a label enables, for example, the binding reaction among the target, the labeled probe, and the carrier-binding probe to be performed in a dispersed state, and, moreover, enables a first bound body to be bound to the carrier through the carrier-binding probe within the first bound body obtained. Thus, in the analysis method of the present embodiment, for example, during a binding reaction in which the labeled probe and the target dispersed in a reaction system (for example, a reaction solution) bind together, the binding reaction occurs with higher probability compared to methods in which the target is analyzed using the carrier to which the labeled probe is immobilized, or to methods in which the target is analyzed using a first bound body resulting from a binding reaction between the carrier-binding probe and the carrier. This enables the binding reaction duration to be shortened, and also enables the sensitivity to be further improved. Moreover, in a method in which the target is analyzed using the carrier to which the labeled probe is immobilized, for example, the label is detected even when the target is not present, and thus background noise is high. However, the analysis method of the present embodiment, for example, enables the label to be detected for the carrier bonded to the first bound body. Thus, in the analysis method of the present embodiment, for example, background noise is lower than in methods in which the target is analyzed using the carrier to which the labeled probe is immobilized.

Specific examples of the binding label include substances in which a functional group capable of binding to the carrier binds specifically to the carrier or to the label on the carrier. The position of the label on the carrier-binding probe is not particularly limited, and may, for example, be a base at the 5' terminal of the probe, a base at the 3' terminal of the probe, or another base, and is preferably a base at the 5' terminal of the probe. The number of binding labels in the carrier-binding probe is not particularly limited, and may be set appropriately according to the type of the binding label.

The length of the carrier-binding probe is not particularly limited, and may be determined appropriately according to the type of the target, for example. In cases in which the target is the target nucleic acid, the length of the carrier-binding probe may be set appropriately according to the length of the target nucleic acid, for example. In cases in which the target nucleic acid is a microRNA, the length of the carrier-binding probe is, for example, from 7 to 25 bases long, and is preferably from 7 to 15 bases long.

The number of binding bases where the target is bound on the labeled probe and the carrier-binding probe is not particularly limited, and may be set appropriately according to the target, for example. In cases in which the target is the target nucleic acid, the number of binding bases of the labeled probe and the carrier-binding probe may be set appropriately according to the length of the target nucleic acid, for example. The number of binding bases of the labeled probe is, for example, from 7 to 15 bases. The number of binding bases of the carrier-binding probe is, for example, from 7 to 25 bases, and is preferably from 7 to 15 bases.

The number of binding bases of the labeled probe may be, for example, less than, more than, or may be equal to the number of binding bases of the carrier-binding probe.

In the analysis method of the present embodiment, the binding positions of the labeled probe and the carrier-binding probe on the target are not particularly limited, and may be different positions, for example. In cases in which the target is the target nucleic acid, the labeled probe may bind, for example, closer to the 5' end side of the target nucleic acid, or may bind closer to the 3' end side of the target nucleic acid, than the carrier-binding probe, and is preferably closer to the 5' end side than the carrier-binding probe. As a specific example, the labeled probe preferably binds to contiguous bases from the 5' end side of the target nucleic acid, and the carrier-binding probe preferably binds to contiguous bases from the 3' end side of the target nucleic acid.

In cases in which the target is the target nucleic acid, the labeled probe may, for example, be a probe formed from a base sequence that is complementary to that of the target nucleic acid, or may be a probe that contains a base sequence complementary to that of the target nucleic acid. In the latter case, the labeled probe contains, for example, an additional sequence and/or a linker. In cases in which the labeled probe contains an additional sequence, for example, the additional sequence of the labeled probe may be labeled by the labeling substance.

In cases in which the target is the target nucleic acid, the carrier-binding probe may, for example, be a probe formed with a base sequence that is complementary to that of the target nucleic acid, or may be a probe that contains a base sequence complementary to that of the target nucleic acid. In the latter case, the carrier-binding probe may, for example, contain an additional sequence and/or a linker. In cases in which the carrier-binding probe contains an additional sequence, for example, the additional sequence of the carrier-binding probe may be labeled by the binding label. The linker is not particularly limited, and, for example, may be a linker of straight or branched alkyl chain of 6 to 16 carbons containing an amide bond, or a linker including a polyethylene oxide structure, and a specific example thereof is a linker formed with an alkyl chain of 6 carbons including an amide bond. In cases in which the labeled probe and the carrier-binding probe contain an additional sequence and/or a linker, the additional sequences and/or linkers of the probes may, for example, be the same as each other, of may be different to each other. Moreover, the lengths thereof may, for example, be the same as each other or may be different to each other.

The base sequence of the additional sequence is not particularly limited. In cases containing the additional sequence, the length of the additional sequence is not particularly limited and is, for example, a length of 1 to 10 bases. Examples of the additional sequence include an RNA and a DNA.

In the analysis method of the present embodiment, the carrier is, for example, a carrier modified by a label (carrier label) capable of binding to the carrier-binding probe. The carrier is not particularly limited and may, for example, be a carrier formed from a light-transmitting material with a specific gravity higher than 1. Specific examples of the carrier include a carrier made from silica, a carrier made from silica gel, a carrier made from agarose, a carrier made from glass (for example borosilicate glass or soda-lime glass), a carrier made from polystyrene, a carrier made from acrylic resin, a carrier made from polyvinyl alcohol resin, or a carrier made from polycarbonate resin. The size of the carrier is not particularly limited. The shape of the carrier is not particularly limited and may, for example, be a spherical shape, such as an ellipse true circle. Specific examples thereof include a spherical bead. In cases in which the carrier is the bead, a lower limit to the diameter of the bead is, for example, 0.1 $\mu$m (100 nm), 200 nm, 250 nm, or 300 nm, and an upper limit thereto is, for example, less than 100 $\mu$m, and more preferably 10 $\mu$m, 5 $\mu$m, 4 $\mu$m, 1 $\mu$m, 800 nm, or 400 nm. The range of diameters of the bead is, for example, from 100 nm to 4 $\mu$m, from 100 nm to 800 nm, from 200 nm to 800 nm, or from 250 nm to 400 nm. Reference here to a light-transmitting material means a material having sufficient light-transmittance when the target is analyzed while concentrated in the detection section.

The method of modifying the carrier using the carrier label is not particularly limited, and any known methods may be employed according to the type of the carrier label. The carrier label is, for example, a label that specifically binds to the carrier-binding probe. Specific examples of the carrier label include a functional group capable of binding to the carrier-binding probe, and a substance that binds specifically to the carrier-binding probe or to the binding label. The number of carrier labels in the carrier is not particularly limited, and may be appropriately set according to the type of the carrier label. The binding between the carrier label and the carrier may be a direct binding or may be an indirect binding. In the latter case, the carrier label binds, for example, to the carrier via the linker.

The binding between the carrier-binding probe and the carrier may, for example, be a direct binding or may be an indirect binding. In the former case, examples of the binding include a binding by a reaction between a functional group of the carrier-binding probe and a functional group of the carrier. In the latter case, examples of the binding include a binding of the carrier-binding probe to the carrier via the binding label, a binding of the carrier to the carrier-binding probe via the carrier label, and a binding between the binding label of the carrier-binding probe and the carrier label of the carrier.

In the analysis method of the present embodiment, it is preferable that the carrier-binding probe includes a first binding substance, that the carrier includes a second binding substance that binds to the first binding substance, and that the carrier-binding probe binds to the carrier through the binding between the first binding substance and the second binding substance. It is sufficient that the first binding substance and the second binding substance are at least a combination of substances in which one of them binds to the other, and, for example, the first binding substance may bind to the second binding substance, or the second binding substance may bind to the first binding substance. The combination of the first binding substance and the second binding substance is not particularly limited, and examples thereof include a combination of biotin and avidin, and a combination of an antigen and an antibody specific to the antigen. Moreover, the first binding substance and the second binding substance may, for example, be opposite combinations to the examples of combinations given above.

In the analysis method of the present embodiment, each of the following processes may, for example, be performed in a reaction system. The reaction system is a liquid system, and the reaction system may, according to the reagents included in the reaction system for example, be described in terms of a first reaction system including the sample, the labeled probe, and the carrier-binding probe, and a second reaction system including the sample, the labeled probe, the carrier-binding probe, and the carrier.

The first reaction process is, as stated above, a process in which the target in the sample, the labeled probe that binds to the target, and the carrier-binding probe that binds to the target and to the carrier contact each other in a liquid solvent, and the target, the labeled probe, and the carrier-binding probe react to bind to each other to form a first bound body. There are no particular limitations to the sequence of the contact and, for example, the labeled probe and the carrier-binding probe may be added to the sample so as to cause the contact. Then the target, the labeled probe, and the carrier-binding probe are caused to bind by, for example, mixing and stirring a mixture obtained after the contact (the first reaction system). The mixing may, for example, be any known methods such as inverting-mixing or vibration. The stirring may, for example, be a method employing a stirring substance. In the first reaction process, the contact conditions between the sample, the labeled probe, and the carrier-binding probe (for example, the temperature, duration, and the like) are not particularly limited. The contact temperature may, for example, be room temperature (about 25°C).

In the first reaction process, the number of labeled probes is not particularly limited and may, for example, be appropriately determined according to the number of targets in the sample. The number of the labeled probes is, for example, the number of the targets or greater. In the first reaction system, the concentration of the labeled probe is not particularly limited and is, for example, from 0.01 nmol/L to 1 nmol/L. The concentration of the labeled probe may, for example, be represented as a concentration of a single labeled probe, or as a total of the concentrations of two types of labeled probe or more (the same applies hereinafter).

In the first reaction process, the number of carrier-binding probes is not particularly limited and may, for example, be appropriately determined according to the number of carrier labels. The number of carrier-binding probes is, for example, the number of the carrier labels or fewer. The concentration of the carrier-binding probe in the first reaction system is, for example, from 0.1 pmol/L to 100 pmol/L. The concentration of the carrier-binding probe may, for example, be represented as a concentration of a single carrier-binding probe, or as a total of the concentrations of two types of carrier-binding probe or more (the same applies hereinafter).

Next, the second reaction process is, as stated above, a process in which the first bound body and the carrier contact each other, and the first bound body and the carrier react to bind to each other to form a second bound body having a specific gravity higher than that of the liquid solvent. The contact sequence is not particularly limited and, for example, the carrier may be added to the first reaction system containing the first bound body to cause the contact to occur. Then, the first bound body and the carrier are caused to bind together via the carrier-binding probe in the first bound body by, for example, mixing and stirring a mixture obtained after the contact (the second reaction system) in a similar manner to in the first reaction process. For the mixing, stirring, and contact conditions (for example, temperature, duration, and the like) in the second reaction process, for example, those described for the first reaction process may be employed.

In the second reaction process the number of the carrier is not particularly limited. In the second reaction system, a volume ratio value (C/R) of the volume (C) of the carrier to the volume (R) of the second reaction system is, for example, from $1/10^2$ to $1/10^5$, and is preferably about $1/10^3$. The volume of the carrier may, for example, be represented as a volume of a single carrier, or may be a total of the volumes of two types of carrier or more (the same applies hereinafter).

The concentration process is, as stated above, a process in which the liquid solvent containing the second bound body is centrifuged to concentrate the second bound body. In the concentration process, for example, the carrier to be concentrated may be the carrier that forms the second bound body, or may be a mixture of the carrier that forms the second bound body and a carrier that does not form the second bound body.

The following analysis process is a process in which the target in the sample is analyzed by detecting, in a concentrated state, a label of the labeled probe contained in the second bound body. The detection of the label is not particularly limited and may, for example, be appropriately determined according to the type of the label. As a specific example, when the label is a fluorescent labeling substance, the detection of the label is, for example, by detection of a fluorescent signal caused by the fluorescent labeling substance. The detection conditions of the fluorescent signal may, for example, be appropriately set according to the type of the fluorescent labeling substance. When the labeled probe is the fluorescent labeled probe, the fluorescent label of the fluorescent labeled probe is, for example as stated above, preferably a substance that undergoes a signal alteration through a binding of the fluorescent labeled probe to the target nucleic acid. In such cases, in the analysis process, for example, the signal alteration of the fluorescent label is detected. As a specific example, when the fluorescent label is a pyrene-containing substance, ultraviolet light is irradiated onto the detection section, and the fluorescent signal caused by the pyrene is detected. The conditions to detect the fluorescent signal caused by the pyrene are, for example, as described above.

The analysis method of the present embodiment may, for example, be implemented by employing a target analyzing chip (also sometimes referred to as an "analyzing chip" hereinafter). It is sufficient that the analyzing chip is, for example, an analyzing chip capable of implementing the first reaction process, the second reaction process, the concentration process, and the analysis process, and the configuration of the analyzing chip is not particularly limited. The analyzing chip is, for example, an analyzing chip including a base plate provided with a reaction section, a detection section, and a flow path communicating the reaction section with the detection section. The reaction section is a location where a target in a sample, a labeled probe that binds to the target, a carrier-binding probe that binds to the target and to a carrier formed from a light-transmitting material, and the carrier react to bind to each other. The detection section is a location where a second bound body is concentrated, the second bound body being formed by a binding between a first bound body, in which the target, the labeled probe, and the carrier-binding probe are bound together, and the carrier via the carrier-binding probe. The flow path includes a hydrophobic internal wall as a movement control means to control movement of the second bound body from the reaction section to the detection section . When a centrifugal force (C) greater than a resistance force (R) arising due to the hydrophobic properties of the flow path is applied, in the liquid solvent, the second bound body having a specific gravity higher than that of the liquid solvent can be moved by the movement control means to the detection section through the flow path. The analysis method of the present embodiment may, for example, be simply implemented by implementation using the analyzing chip. The analyzing chip is, for example, capable of effectively implementing the binding reactions in the reaction processes, and the concentration in the concentration process. The analysis method of the present embodiment is accordingly capable of achieving even more excellent sensitivity and precision due to being implemented by employing the analyzing chip. The description of the target analyzing chip of the present embodiment, described later, may, for example, be employed for the analyzing chip.

When the analyzing chip is employed, the analysis method includes, for example: a process in which the sample is introduced into the reaction section (sample introduction process); a process in which the target in the sample, the labeled probe, and the carrier-binding probe contact each other, and the target, the labeled probe, and the carrier-binding probe react to bind to each other to form a first bound body (first reaction process); a process in which the carrier is introduced into the reaction section (carrier introduction process); a process in which the first bound body and the carrier contact each other and the first bound body and the carrier react to bind to each other to form a second bound body (second reaction process); a process in which the second bound body in the reaction section is moved to the detection section by a centrifugal force (C) greater than a resistance force (R) arising due to the hydrophobic properties, and the carrier is concentrated (concentration process); and a process in which the target in the sample is analyzed in the detection section by detecting a label of the labeled probe bound to the concentrated carrier (analysis process).

The sample introduction process is, for example as described above, a process in which the sample is introduced into the reaction section. The method of introducing the sample is not particularly limited. The sample is, for example, introduced through an opening in the reaction section. In the reaction section, as described later, in case that the labeled probe and the carrier-binding probe are immobilized, the labeled probe and the carrier-binding probe are released from their immobilization and freed by the introduction of the sample. Moreover, in case that the labeled probe and the carrier-binding probe are not immobilized, a process may be provided in which, at the same time as the sample introduction process, or at the time therebefore or thereafter, the labeled probe and the carrier-binding probe are introduced to the reaction section.

The first reaction process is, for example, a process in which the target in the sample, the labeled probe, and the carrier-binding probe contact each other, and the target, the labeled probe, and the carrier-binding probe react to bind to each other to form a first bound body. At this process, the mixture in the reaction section of the sample, the labeled probe, and the carrier-binding probe is preferably stirred. The stirring method is not particularly limited.

The stirring method is, for example, a method employing the stirring substance. In such cases, the reaction section of the analyzing chip preferably further includes the stirring substance, and the mixture in the reaction section is stirred by the stirring substance. The stirring using the stirring substance may, for example, be performed by placing a magnet outside the analyzing chip and moving the stirring substance with the magnet. The stirring substance may, for example, be caused to spin, may be caused to orbit, or may be moved randomly.

Moreover, a method employing a centrifuge may, for example, be employed as the stirring method. The analyzing chip may, as described later, be employed, for example, by placing on a centrifuge so as to utilize centrifugal force to move the carrier from the reaction section to the detection section. Thus, the stirring may be performed in the reaction processes by centrifuging the analyzing chip. The centrifuging may, for example, be centrifuging in a single direction, or may be centrifuging by rotating alternately in opposite directions.

The carrier introduction process is, for example, a process in which the carrier is introduced into the reaction section. The method of introducing the carrier is not particularly limited. The carrier is, for example, introduced through an opening in the reaction section.

Note that the carrier may be immobilized in the reaction section in advance. In such cases, the carrier introduction process may be omitted.

The second reaction process is, for example, a process in which the first bound body and the carrier contact each other, and the first bound body and the carrier react to bind to each other to form a second bound body. At this process, a mixture of the first bound body and the carrier is preferably stirred in the reaction section. The stirring method may, for example, be the stirring method described above.

The following concentration process is, for example, a process in which the second bound body in the reaction section is moved to the detection section by centrifugal force (C) greater than the resistance force (R) arising due to the hydrophobic properties, and the carrier is concentrated. In the concentration process, the concentrated carrier may, for example as described above, be carrier that forms the second bound body, or may be a mixture of carrier that forms the second bound body and carrier that does not form the second bound body.

The resistance force (R) may, for example, be appropriately calculated according to the degree of hydrophobicity of the inner walls of the flow path. As a specific example, the resistance force (R) may, for example, be calculated from a surface tension of a mixture including the sample, the reagents, and the carrier, a contact angle between the inner walls of the flow path and a mixture including the sample, the reagents, and the carrier, the width and height of the flow path of the analyzing chip, and the like, as specifically expressed by Equation (1) below. In cases in which the contact angle ($\theta$) is 90° or greater between the inner walls of the flow path, and the mixture including the sample, the reagents, and the carrier, the resistance force (R) can, for example, be favorably calculated according to Equation (1) below.

$$R = -2wh\gamma\cos\theta \ (1/w + 1/h) \qquad \ldots(1)$$

R: resistance force (N)
$\gamma$: surface tension of a mixture including sample, reagents, and carrier (N/m)
$\theta$: contact angle between the inner walls of the flow path, and a mixture including sample, reagents, and carrier
w: width of flow path (m)
h: height of flow path (m)

The centrifugal force (C) is not particularly limited as long as it is, for example, capable of moving the second bound body to the detection section. In the analysis method of the present embodiment, the mixture of the sample, reagents, and carrier is, for example, introduced into the flow path by the centrifugal force (C). Moreover, the carrier in the mixture (for example, the second bound body) is, for example, accumulated in the detection section of the analyzing chip at the opposite end side of the flow path by the centrifugal force (C). Note that the duration ($\Delta t$) required to accumulate the carrier (the second bound body) in the detection section may, for example, be calculated from the viscosity and density of the mixture, the density of the carrier, the radius of the carrier, the speed of revolution during centrifuging, the start point and the end point on the rotation radius, and the like, as specifically expressed according to Equation (2) below. For example, Equation (2) below enables the density and radius of the carrier and the centrifugal force (C), namely the rotation radius and rotation speed (angular speed of rotation) in order to accumulate the carrier in the detection section within a desired duration of centrifuging to be calculated (or estimated).

$$\Delta t = [18\mu/(4\omega^2 R^2( \rho_s - \rho_l))]\ln(r_2/r_1) \qquad \ldots(2)$$

$\Delta t$: time needed to move the carrier from a start point $r_1$ to an end point $r_2$ of rotation radius (seconds)
$\mu$: viscosity of mixture of sample, reagents, and carrier (Pa·sec)
$\rho_1$: density of mixture of sample, reagents, and carrier (kg/m$^3$)
$\rho_s$: density of carrier (kg/m$^3$)
R: radius of carrier (m)
$\omega$: angular speed of rotation (rad/sec)
$r_1$: start point of rotation radius (minimum distance from axis to reaction section during centrifuging) (m)
$r_2$: end point of rotation radius (maximum distance from axis to detection section) (m)

In the analysis method of the present embodiment, the centrifugal force on the analyzing chip may, for example, be imparted by employing a centrifuge. In such cases, the analyzing chip, for example, preferably further includes a placement position on the centrifuge. Then, for example, the analyzing chip is placed in the centrifuge at the placement position, and centrifugal force can be applied to the analyzing chip by the centrifuge.
The analysis process is, for example as described above, a process in which the target in the sample is analyzed by detecting a label of the labeled probe bound to the concentrated carrier, in the detection section. The detection of the label may, for example, be executed as described above.

Target Analyzing Chip

The target analyzing chip of the present embodiment (sometimes referred to as the "analyzing chip" hereinafter) includes, as described above, a base plate provided with a reaction section, a detection section, and a flow path communicating the reaction section with the detection section. The reaction section is a location where a target in a sample, a labeled probe that binds to the target, a carrier-binding probe that binds to the target and to a carrier formed from a light-transmitting material and the carrier react to bind to each other. The detection section is a location where a second bound body is concentrated, the second bound body being formed by a binding between a first bound body, in which the target, the labeled probe, and the carrier-binding probe are bound together, and the carrier via the carrier-binding probe. The flow path includes a hydrophobic internal wall as a movement control means to control movement from the reaction section to the detection section of the second bound body. When a centrifugal force (C) greater than a resistance force (R) arising due to the hydrophobic properties of the flow path is applied, in the liquid solvent, the second bound body having a specific gravity higher than that of the liquid solvent can be moved by the movement control means to the detection section through the flow path.

In the analyzing chip of the present embodiment, due to the flow path including the internal wall with hydrophobic properties as the movement control means, the movement of the carrier from the reaction section to the detection section can be controlled. This means that, for example, capillary action from the reaction section to the detection section is generated by a predetermined centrifugal force, without such capillary action occurring spontaneously. Thus, the analyzing chip of the present embodiment may, for example, be described as a chip that generates a capillary action from the reaction section to the detection section by a predetermined centrifugal force, without such capillary action occurring spontaneously. In the analyzing chip of the present embodiment, the carrier whose movement from the reaction section to the detection section is controlled may, for example, be one out of a carrier that forms the second bound body or a carrier that does not form the second bound body, or may be a mixture thereof, and is preferably a mixture thereof. The analyzing chip of the present embodiment is a chip that is employed for the analysis method of the present embodiment, and the descriptions already given above of the analysis method of the present embodiment may be employed therefor.

In the target analyzing chip of the present embodiment, the carrier may be pre-fixed to the reaction section of the base plate, or, moreover, may be packaged with the base plate by packing individually. In the latter case, the target analyzing chip of the present embodiment may, for example, be referred to as a target analyzing kit.

In the analyzing chip of the present embodiment, the reaction section and the detection section are, for example, disposed in sequence along one direction. In the analyzing chip of the present embodiment, in a state in which the analyzing chip is mounted on a horizontal plane, a direction that is parallel to the horizontal plane and runs from the reaction section toward the detection section is also referred to as the "length direction", or the "movement direction" of the sample and the carrier. A direction that is parallel to the horizontal plane and is perpendicular to the length direction is also referred to as the "width direction". Moreover, a direction that is perpendicular to the horizontal plane (a perpendicular direction to the length direction and the width direction) is also referred to as the "thickness direction" or the "depth direction". In the analyzing chip of the present embodiment, the carrier is also referred to as the "analysis carrier" hereinafter.

In the analyzing chip of the present embodiment, the base plate may, for example, include a lower plate and an upper plate. In such cases, for example, the reaction section, the flow path, and the detection section are preferably formed by stacking the lower plate and the upper plate.

A first specific example is, for example, given as a configuration in which an upper surface of the lower plate (the surface stacked against the upper plate) is flat, the upper plate includes a through hole that becomes an opening through which the sample is introduced to the reaction section, a lower surface of the upper plate (the surface stacked against the lower plate) includes a recess that will become the reaction section, the flow path, and the detection section, and the through hole and the recess is connected together. In such cases, by stacking the lower plate and the upper plate together, the opening is formed by the through hole of the upper plate and the upper surface of the lower plate, and the reaction section, the flow path, and the detection section are formed in communication with each other by the recess of the upper plate and the upper surface of the lower plate.

A second specific example is, for example, given as a configuration in which a lower surface of the upper plate (the surface stacked against the lower plate)is flat, the upper plate includes a through hole that becomes an opening through which the sample is introduced to the reaction section, the upper surface of the lower plate (the surface stacked against the upper plate) includes a recess that will become the reaction section, the flow path, and the detection section; and the through hole and a portion of the recess corresponding to the reaction section are positioned in the same plane as each other. In such cases, by stacking the lower plate and the upper plate together, the opening is formed by the through hole of the upper plate, and the reaction section, the flow path, and the detection section are formed in communication with each other by the recess of the lower plate and the through hole and lower surface of the upper plate.

In the analyzing chip of the present embodiment, the size and shape of the reaction section is not particularly limited.

An embodiment of the reaction section has, for example, a configuration in which a bottom face of the reaction section is formed by the upper surface of the lower plate, and an upper face of the reaction section is formed by the lower surface of the upper plate. The reaction section is, for example, a hollow pillar shape (also referred to as a tube shape), and specifically is preferably a pillar shape having an axial direction in the thickness direction of the analyzing chip. The pillar shape is, for example, a circular pillar shape, an angular pillar shape, or the like. The reaction section may, for example, include an air hole, and the air hole may, for example, be formed as a through hole in the upper plate at an upper face side of the reaction section.

In the analyzing chip of the present embodiment, an embodiment of the flow path has, for example, a configuration in which the bottom face of the flow path is formed by the upper surface of the lower plate, and the upper face of the flow path is formed by the lower surface of the upper plate. The flow path is hollow, and specifically, is a hollow having an axial direction that is the length direction of the analyzing chip. In cross-section perpendicular to the axial direction (i.e., cross-section of the hollow), the flow path has a circular shape, such as a true circle, ellipse, or the like, or has a polygonal shape, such as a square, rectangle, or the like.

The flow path, for example, preferably includes a movement promotion means to promote movement of the analysis carrier from the reaction section side toward the detection section side. The presence of the movement promotion means enables, for example, the efficient movement of the analysis carrier, and the efficient recovery of the analysis carrier in the detection section. The movement promotion means in the flow path is not particularly limited and may, for example, have a structure in which the cross-sectional area decreases on progression from the reaction section side to the detection section side, as described later.

The flow path is, for example, a structure that has a cross-sectional area that decreases on progression from the reaction section side to the detection section side. The structure can, for example, be a structure that narrows on progression from the reaction section side to the detection section side. The cross-sectional area of the flow path is, for example, an area of cross-section perpendicular to the length direction of the analyzing chip, and an area of cross-section of the hollow on the flow path.

The cross-sectional area may, for example, decrease continuously, or may decrease discontinuously (or stepwise) on progression from the reaction section side to the detection section side. As a specific example, the flow path may, for example, have a cross-section profile parallel to the axial direction that narrows in a tapered profile on progression from the reaction section side toward the detection section side. Specifically, the flow path may, for example, have a cross-section profile as viewed from a side face of the analyzing chip that is a profile that narrows in a tapered profile on progression from the reaction section side to the detection section side, may have a cross-section profile as viewed from the upper surface (or the lower surface) of the analyzing chip that is a profile that narrows in a tapered profile on progression from the reaction section side to the detection section side, or both.

The side faces of the flow path are, for example, preferably curved faces that narrow in a tapered profile on progression from the reaction section to the detection section side. Adopting such a profile for the side faces of the flow path enables the efficient movement of the analysis carrier, and the efficient recovery of the analysis carrier in the detection section. In order to enable the analysis carrier to be recovered at the detection section with greater efficiency, the radius of curvature R of the curved face is, for example, from 1 mm to 5 mm, from 1.5 mm to 4.5 mm, or approximately 3 mm (for example, from 2.5 mm to 3.5 mm). The radius of a curvature R of the curved face is, for example, the radius of the curvature of the curved face that narrow with the tapered profile.

As stated above, the flow path includes a movement control means to control movement of the analysis carrier from the reaction section to the detection section. The second bound body in the reaction section can be moved through the flow path to the detection section by the movement control means when the second bound body in the reaction section has a specific gravity higher than that of the liquid solvent, and a centrifugal force (C) greater than a resistance force (R) arising due to the hydrophobic properties of the flow path is applied thereto. Specifically, in cases in which the flow path includes an internal wall with hydrophobic properties as the movement control means, for example, capillary action from the reaction section to the detection section does not occur spontaneously when a centrifugal force (C) greater than the resistance force (R) arising due to the hydrophobic properties of the flow path on the second bound body is not applied, thereby enabling the second bound body to be retained in the reaction section. Moreover, due to capillary action from the reaction section to the detection section occurring when a centrifugal force (C) greater than the resistance force (R) is applied to the second bound body, the second bound body can be moved from the reaction section to the detection section. Thus, in the analyzing chip of the present embodiment, the hydrophobic properties, for example, refer to hydrophobic properties of an extent such that movement of a liquid fraction from the reaction section to the detection section by capillary action does not occur spontaneously. In the present embodiment, in addition to the second bound body, the analysis carrier that does not form the second bound body may also be capable of moving to the detection section through the flow path when, for example, the centrifugal force (C) is applied thereto. The internal wall with hydrophobic properties of the flow path may, for example, be formed by a hydrophobic member, as described later. In the flow path, for example, all the internal walls on the upper and lower faces and side faces may have hydrophobic properties, or some of the internal walls may have hydrophobic properties. As a specific example, when the analyzing chip is formed by an upper plate and a lower plate, for example, the inner walls of the flow path formed by the upper plate may have hydrophobic properties, the inner walls of the flow path formed by the lower plate may have hydrophobic properties, or the inner walls of the flow path formed by the upper plate and the lower plate may have hydrophobic properties. Furthermore, in the analyzing chip of the present embodiment, inner walls of the detection section may have hydrophobic properties, and the movement control means may include the hydrophobic inner walls of the detection section. The descriptions already given above may be employed for the resistance force (R) and the centrifugal force (C).

The size of an internal space of the flow path is not particularly limited. A height (depth) of the flow path is, for example, from 1 $\mu$m to 3,000 $\mu$m, and is preferably from 100 $\mu$m to 500 $\mu$m. The width of the flow path is, for example, from 1 $\mu$m to 3,000 $\mu$m, and is preferably from 100 $\mu$m to 500 $\mu$m. The height and width of the flow path are, for example, the height and width of the internal space of the flow path.

In the analyzing chip of the present embodiment, the profile of the detection section is not particularly limited. The detection section, for example, may be a hollow extension of the flow path. Namely, in the analyzing chip of the present embodiment, the detection section may, for example, be an end region of the flow path communicating with the reaction section, on the opposite side of the flow path to the reaction section. The profile of the detection section is not particularly limited, and a cross-section perpendicular to the axial direction (for example, a cross-section of the hollow) has, for example, a circular shape, such as a true circle, ellipse, or the like, or a polygonal shape, such as a square, rectangle, or the like.

The capacity of the internal space of the detection section is, for example, preferably smaller than a volume of the sample. By designing the capacity of the detection section, for example, to be relatively small compared to the sample in this manner, the concentration of the analysis carrier in the detection section can be set relatively high. The capacity of the internal space of the detection section is, for example, from $10^{-2}$ times to $10^{-6}$ times (for example, $10^{-4}$ times) as much as the volume of the sample. Moreover, a lower limit to the capacity of the detection section, for example, preferably exceeds a volume of the total amount of the analysis carrier disposed in the reaction section. The capacity of the internal space of the detection section, for example, is preferably smaller than the capacity of the internal space of the reaction section. By designing the capacity of the detection section, for example, to be relatively small compared to the capacity of the internal space of the reaction section in this manner, the concentration of the analysis carrier in the detection section can be set relatively high. The capacity of the internal space of the detection section is, for example, from $10^{-1}$ times to $10^{-6}$ times, and preferably from $10^{-2}$ times to $10^{-5}$ times (and more preferably, $10^{-4}$ times) as much as the capacity of the internal space of the reaction section. The height (depth) of the detection section is not particularly limited and is, for example, from 1 $\mu$m to 3,000 $\mu$m, from 10 $\mu$m to 300 $\mu$m, or is approximately 100 $\mu$m (preferably from 50 $\mu$m to 150 $\mu$m). Setting the height of the detection section in this manner, for example, increases a proportion of the carrier moved to the detection section for which a label is detectable. The width of the detection section is not particularly limited and is, for example, from 1 $\mu$m to 3,000 $\mu$m, and is preferably from 10 $\mu$m to 300 $\mu$m. The height and width of the detection section are, for example, a height and width of the internal space of the detection section, respectively. When the height of the detection section is from 1 $\mu$m to 500 $\mu$m, the proportion of the carrier that moves to the detection section is improved, and the proportion for which the label is detectable is further increased. Therefore, the diameter of the carrier is, for example, from 100 nm to 4 $\mu$m, from 100 nm to 800 nm, or is preferably from 250 nm to 400 nm. Setting the height (depth) of the detection section in this manner enables even a carrier such as a silica beads to have light-transmitting properties in the detection section.

In the analyzing chip of the present embodiment, as stated above, the reaction section contains the labeled probe that binds to the target, and the carrier-binding probe that binds to the target and the carrier. The descriptions of the labeled probe and the carrier-binding probe may, for example, employ the descriptions already given above.

In the analyzing chip of the present embodiment, the labeled probe and the carrier-binding probe may, for example, be in a free state in the reaction section. Moreover, the labeled probe and the carrier-binding probe may, for example, be in an immobilized state so as to be capable of being freed in the reaction section (on the inner walls of the reaction section). In the latter case, the labeled probe and the carrier-binding probe are, for example, preferably immobilized by a water soluble material in the reaction section. In such cases, for example, the labeled probe and the carrier-binding probe are freed from the reaction section (the inner walls of the reaction section) by the sample introduced through the opening to the reaction section. The water soluble material is, for example, polyvinyl alcohol, cellulose derivatives such as carboxymethyl cellulose or methylcellulose, a water soluble polymer such as polyacrylic acid-based polymers, polyacrylamide, polyethylene oxide, starch, or gelatin, an oligosccharide such as sucrose, trehalose, mannitol, or lactose. Note that the analysis carrier may also, similarly to the foregoing, be in an immobilized state so as to be capable of being freed in the reaction section (on the inner walls of the reaction section).

In the analyzing chip of the present embodiment, for example, as well as the labeled probe and the carrier-binding probe, other substances as reagents may also be included in the reaction section. Such other substances are not particularly limited, and include, for example, surfactants, buffers, salts, water soluble macromolecules, and sugars. The surfactant is, for example, a non-ionic surfactant, an ionic surfactant, or the like, and is preferably a non-ionic surfactant in order to promote a reaction between the target and the labeled probe. The non-ionic surfactant is, for example, Tween 20 (registered trademark), Triton (trademark) X-100, sucrose fatty acid ester, sorbitan fatty acid ester, polyoxyethylene sorbitan fatty acid ester, fatty acid alkanolamide, polyoxyethylene alkyl ether, polyoxyethylene alkylphenyl ether, or the like. The other substances may, for example, be included together with the analysis carrier.

In the analyzing chip of the present embodiment, the reaction section may further include a stirring substance. In such cases, a mixture liquid of the sample and the reagents disposed in the reaction section, or a mixture liquid of the first bound body and the analysis carrier, can be stirred by the stirring substance in the reaction section, enabling the target, the labeled probe and the carrier-binding probe in the sample, or the first bound body and the analysis carrier, to efficiently contact each other. A magnetic stirring substance can, for example, be employed as the stirring substance, and a specific example thereof is, for example, a stirrer such magnetic beads. The material of the stirring substance is not particularly limited, and, for example, is preferably a magnetic body such as SUS. The shape of the stirring substance is, for example, spherical, and the size thereof is, for example, a diameter of from 0.1 mm to 5 mm (for example, 1 mm).

In the analyzing chip of the present embodiment, the material of the base plate is not particularly limited and, for example, a glass such as quartz glass or borosilicate glass, or a resin such as polydimethylsiloxane (PDMS), polystyrene, an acrylic resin, a cycloolefin based resin, polyethylene terephthalate, or the like may be employed therefor, and a hydrophobic member, as described later, is preferably employed therefor. Moreover, in cases in which the base plate includes a lower plate and an upper plate, as described above, the two plates may, for example, be formed by the same material, or by different materials.

In the analyzing chip of the present embodiment, the detection section is, for example, formed from a light-transmitting member, and is preferably an ultraviolet light-transmitting member. The light-transmitting member is, for example, a glass such as quartz glass or borosilicate glass, a resin such as PDMS, or the like.

In the analyzing chip of the present embodiment, an inner wall of the flow path has hydrophobic properties, as described above. The internal wall having hydrophobic properties may, for example, be configured by employing a hydrophobic member as the base plate, may be configured by laminating a hydrophobic member on a surface of the base plate, or may be configured by modifying a surface of the base plate so as to have hydrophobic properties. Examples of the hydrophobic member include, for example, a hydrophobic resin such as polydimethylsiloxane (PDMS), polystyrene, an acrylic resin, a cycloolefin based resin, or the like. Moreover, the method of modifying to have hydrophobic properties is not particularly limited, and any known methods may be employed therefor.

Since the analyzing chip of the present embodiment is, as described above for example, placed on a centrifuge and applied with a centrifugal force, the analyzing chip preferably further includes a site for placement on the centrifuge.

The sample to be introduced into the analyzing chip of the present embodiment is a sample containing the target; however, the present embodiment is not limited thereto. The sample may, for example, be a sample that does not contain the target, and may be a sample in which it is not known whether a target is contained or not. Embodiments of the sample are not particularly limited, and, for example, the embodiments described above may be employed therefor.

The amount of the sample to be introduced into the analyzing chip of the present embodiment is not particularly limited, and may, for example, be from 1 $\mu$L to 100 $\mu$L (for example, 50 $\mu$L) per single analyzing chip.

A description follows, with reference to the drawings, of specific examples of a target analyzing chip of the present embodiment and a target analysis method of the present embodiment employing the same in which a fluorescent labeled probe is employed as the labeled probe and a target RNA as the target is analyzed. Note that the present embodiment is not limited to these examples.

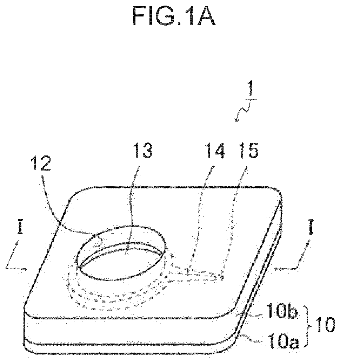

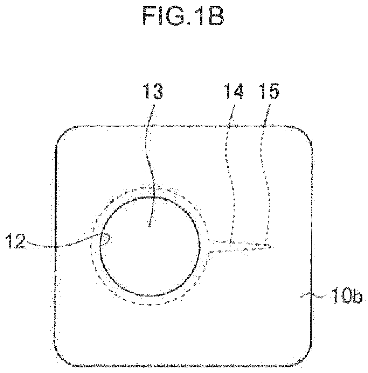

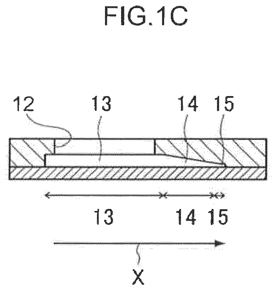

Figs. 1A to 1C are schematic diagrams illustrating an example of an analyzing chip of the present embodiment. Fig. 1A is a perspective view, Fig. 1B is a plan view, and Fig. 1C is a cross-section taken along the direction of I-I of Fig. 1A.

As illustrated in Figs. 1A to 1C, an analyzing chip 1 includes a base plate 10 configured by a lower plate 10a and an upper plate 10b. The upper plate 10b includes a through hole 12, and recesses 13, 14, 15 in a lower surface of the upper plate 10b. Due to stacking the upper plate 10b and the lower plate 10a together, the through hole 12 configures an opening 12 of a reaction section, the recess 13 configures a reaction section 13, the recess 14 configures a flow path 14, and the recess 15 configures a detection section 15. The reaction section 13 and the detection section 15 are in communication with each other through the flow path 14. The arrow X in Fig. 1C indicates a movement direction of a sample in the analyzing chip 1 (length direction of the analyzing chip 1).

The size of the analyzing chip 1 is not particularly limited, and the following parameters are examples thereof.

Recess 13

Diameter: from 1 mm to 30 mm (for example 8 mm)
Volume of introduced sample: from 1 $\mu$L to 1,000 $\mu$L (for example, 50 $\mu$L) Flow Path 14
Length: from 10 $\mu$m to 5,000 $\mu$m (for example, 2,500 $\mu$m)
Width at an end of reaction section 13: from 100 $\mu$m to 5,000 $\mu$m (for example, 1,000 $\mu$m)
Depth at the end of reaction section 13: from 100 $\mu$m to 5,000 $\mu$m (for example, 2,000 $\mu$m)
Width at an end of detection section 15: from 10 $\mu$m to 1,000 $\mu$m (for example, 150 $\mu$m)
Depth at the end of detection section 15: from 10 $\mu$m to 1,000 $\mu$m (for example, 150 $\mu$m)

Detection Section 15

Length: from 10 $\mu$m to 1,000 $\mu$m (for example, 220 $\mu$m)

Width: from 10 $\mu$m to 1,000 $\mu$m (for example, 150 $\mu$m)
Depth: from 10 $\mu$m to 1,000 $\mu$m (for example, 150 $\mu$m)
Capacity: from 0.001 nL to 1,000 nL (for example, 5 nL)

As illustrated in Figs. 1A to 1C, the flow path 14 has a narrowing shape that narrows on progression from the reaction section 13 side toward the detection section 15 side. Specifically, as viewed from the upper surface of the analyzing chip 1, as illustrated in Fig. 1B, both sides of the flow path 14 narrow in a tapered profile on progression from the reaction section 13 side toward the detection section 15 side. Moreover, as viewed from a side face of the analyzing chip 1, as illustrated in Fig. 1C, an upper face of the flow path 14 is formed in a shape that narrows with a tapered profile on progression from the reaction section 13 side toward the detection section 15 side.

The analysis carrier (not illustrated in the drawings) is disposed in the reaction section 13 of the analyzing chip 1. The analysis carrier is preferably immobilized by the water soluble material as described above.

Analysis of RNA in the sample used in the analyzing chip 1 may, for example, be performed in the following manner. The embodiment mentioned below is an example of a case in which the fluorescent label of the fluorescent labeled probe is a pyrene-containing substance, and a stirrer, serving as a magnetic stirring substance and made from an SUS bead having a diameter of 1 mm, is similarly immobilized in the reaction section 13 using a water soluble material.

First, the sample is introduced into the reaction section 13 of the analyzing chip 1 through the opening 12. The analysis carrier and the magnetic stirring substance that are immobilized in the reaction section 13 by the water soluble material are then freed by the liquid solvent of the sample. The sample and the analysis carrier then contact each other in the reaction section 13 by spinning the magnetic stirring substance in the reaction section 13 using a magnet disposed outside the analyzing chip 1. The target RNA in the sample and the fluorescent labeled probe on the analysis carrier are thereby bound together.

Next, the analyzing chip 1 is centrifuged, and the analysis carrier is moved by centrifugal force C from the reaction section 13 to the detection section 15 through the flow path 14. Then, in the detection section 15, ultraviolet light (for example, 340 nm) is irradiated onto the analyzing chip 1 from above, and pyrene-induced fluorescence generated by the binding of target RNA to the fluorescent labeled probe is detected at a predetermined wavelength (for example, 480 nm) by a light detector. The pyrene-modified fluorescent labeled probe generates fluorescence only in a state bound to the target RNA, and thus the amount (or the absence) of the pyrene-induced fluorescence exhibits a correlation with the amount (or the absence) of the target RNA. Accordingly, qualitative analysis or quantitative analysis of the target RNA can be performed by detecting the fluorescence.

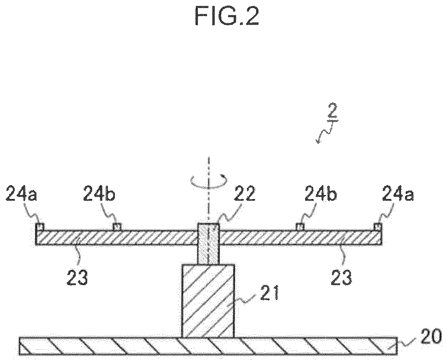

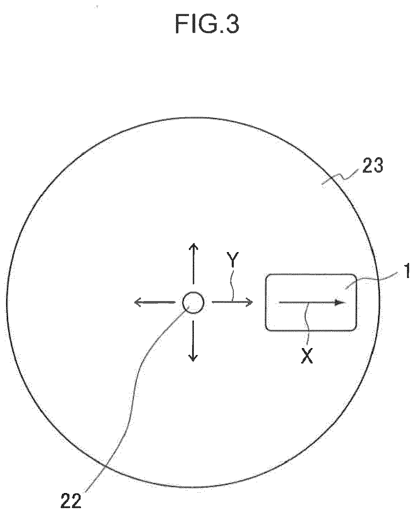

In the present embodiment, as described above, the centrifugal force on the analyzing chip can, for example, be achieved by using a centrifuge. Fig. 2 and Fig. 3 are schematic diagrams illustrating an example of a centrifuge.

Fig. 2 is a cross-section of a centrifuge. A centrifuge 2 includes a base 20, a motor 21, a shaft 22, and a stage 23. The stage 23 includes fixing portions 24a, 24b to fix the disposed analyzing chip. Fig. 3 is a plan view of an upper face of the stage 23 of the centrifuge 2.

Placement of the analyzing chip on the centrifuge 2 is performed, for example, as follows. First, the analyzing chip 1 is placed on the stage 23. Here, the analyzing chip 1 is disposed such that the reaction section 13 side (see Fig. 1) locates near the shaft 22 side. Then, the analyzing chip 1 is fixed to the stage 23 by the fixing portions 24a, 24b. Next, the motor 21 is driven in order to rotate the stage 23 around the shaft 22. Centrifugal force is thereby applied in the arrow Y direction from the reaction section 13 side toward the detection section 15 side of the analyzing chip 1, enabling movement of the sample or movement of the analysis carrier to be performed in the arrow X direction of the analyzing chip 1.

Examples

Explanation follows regarding Examples of the present embodiment. Note that the present invention is not limited to the Examples below.

Example 1

The analysis method of the present embodiment was confirmed to be capable of analyzing target RNA.

(1) Preparation and Evaluation of Avidin-Modified Beads

Two hundred $\mu$L of a 10 mmol/L aqueous HCl solution was added to 20 $\mu$L of an undiluted solution of NHS-activated Sepharose beads (NHS-activated Sepharose 4 Fast Flow, manufactured by GE Healthcare Company) and then stirred. After the stirring, the mixed solution was centrifuged at 2840 g, and 200 $\mu$L of supernatant was removed. The beads were then further washed by adding an aqueous HCl solution to the obtained precipitate and centrifuged twice.

One hundred and eighty μL of avidin binding-reaction solution was added to the washed beads. The avidin binding-reaction solution was an aqueous solution containing 0.8 μmol of avidin, 0.2 mol/L of NaHCO$_3$, and 0.5 mol/L of NaCl. The obtained reaction solution after adding the avidin binding-reaction solution was then stirred for 14 hours at 4°C. The reaction solution was then centrifuged at 2840 g, and 180 μL of supernatant was removed. To the obtained precipitate was then further added 180 μL of blocking solution. The blocking solution was an aqueous solution containing 0.2 mol/L of 2-aminoethanol (ethanolamine), 0.2 mol/L of NaHCO$_3$, and 0.5 mol/L of NaCl. The obtained reaction solution after adding the blocking solution was then stirred for 30 minutes at 20°C. After the stirring, the reaction solution was centrifuged at 2840 g, and 180 μL of supernatant was removed.

Three hundred and eighty μL of a 10 mmol/L phosphate buffer solution (PBS, pH 7.0) containing 0.1 mol/L of NaCl was added to the obtained precipitate. Then after centrifuged at 2840 g, 380 μL of supernatant was removed. The beads were washed by adding the phosphate buffer solution and centrifuged twice. μL of phosphate buffer solution was added to the washed beads, and 400 μL of an avidin-modified bead dispersion was thus prepared. The concentration of avidin-modified beads in the bead dispersion was about 25 particles/μL.

One hundred and fifty μL of an 0.67 mol/L aqueous solution containing biotin-modified DNA was added to 50 μL of the bead dispersion. The mixed solution after adding the aqueous solution was then stirred for 30 minutes at 20°C. The supernatant was then recovered after further centrifuged at 2,840 g. An absorbance photometer (UV1800, manufactured by Shimadzu Corporation) was employed to measure the spectrum of the supernatant. The spectrum of the biotin-modified DNA solution was also measured in a similar manner. Based on the spectrum of the supernatant and the biotin-modified DNA solution, the number of moles of biotin capable of binding to one particle of the avidin-modified bead was calculated using a regular method. This gave a result of about 40 fmol for the number of moles of biotin capable of binding to one particle of the avidin-modified bead. Such avidin-modified beads were used in the analysis described later.

(2) Nucleic Acid Reagent

Target RNA molecules formed with a base sequence given in sequence number 1 below were synthesized as the target RNA. A pyrene-labeled probe formed with a base sequence given in sequence number 2 below was also synthesized as the fluorescent labeled probe. The pyrene-labeled probe was synthesized using 2'-O-methyl RNA. In the base sequence shown as sequence number 2 below, cytidine with a pyrene label on a 2' hydroxyl group was employed for the underlined "n's". A biotinylated probe was also synthesized as a carrier-binding probe with a base sequence given in sequence number 3 below in which a 5' terminal thereof was biotinylated. Note that in the biotinylated probe, four bases from the 5' end is an additional sequence. The pyrene-labeled probe binds to fourteen contiguous bases from a 5' end of the target RNA molecule, and the biotinylated probe binds to bases of the target RNA molecule to which the pyrene-labeled probe does not bind.

Target RNA model (sequence number 1): 5'- agucaauagggugugugagagacuuaacug -3'
Pyrene-labeled probe (sequence number 2): 5'- acacnnuauugacu -3'
Biotinylated probe (sequence number 3): 5'- cagccagttaagtctctcac -3'

(3) Analysis

One hundred and twenty μL of 12.5 mmol/L PBS (pH 7.00) was prepared containing 1.25 μmol/L of the target RNA molecule, 1.25 mol/L of the pyrene-labeled probe, and 1.25 mol/L of the biotinylated probe. The reaction solution obtained after the preparation was incubated for 4 hours at 15°C to bind the target RNA molecule, the pyrene-labeled probe and the biotinylated probe to each other. Next, 40 μL of the bead dispersion was added to the reaction solution, and the mixed solution obtained was stirred for 1 hour at 15°C, thereby binding a first bound body, which was formed from the target RNA molecule, the pyrene-labeled probe, and the biotinylated probe, to the avidin-modified beads.

The mixed solution after binding was then centrifuged at 2840 g, and the supernatant was recovered. The precipitate after centrifuged was then dispersed in a portion of the supernatant. One hundred μL of the obtained dispersion was dripped into a glass bottom dish (manufactured by Matsunami Glass Ind., Ltd.). A fluorescent signal from the avidin-modified beads on the glass bottom dish was then measured using a phase-contrast/fluorescence microscope (ECLIPSE TE300, made by Nikon Corporation). Note that the fluorescent signal was measured at 400 or above nm under excitation light of 340 nm ± 10 nm. The exposure duration for measurement photos was set at 2 seconds. Comparative Examples 1A, 1B and 1C were also measured that were similar thereto, except in that the biotinylated probe was not added for Comparative Example 1A, that the target RNA molecule was not added for Comparative Example 1B, and the pyrene-labeled probe was not added for Comparative Example 1C. A fluorescent signal was also measured for Comparative Example 1D that was similar thereto, except in that the biotinylated probe, the target RNA molecule, and the pyrene-labeled probe were not added.

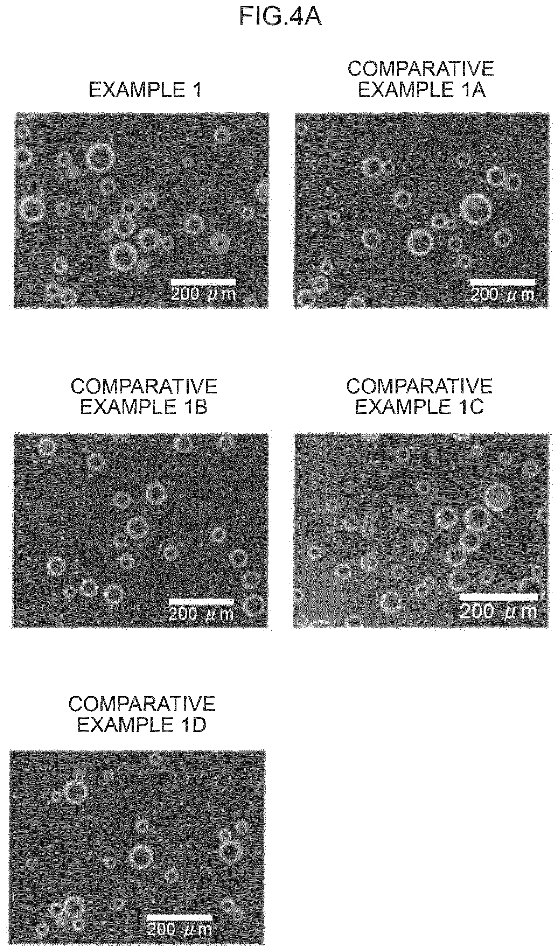

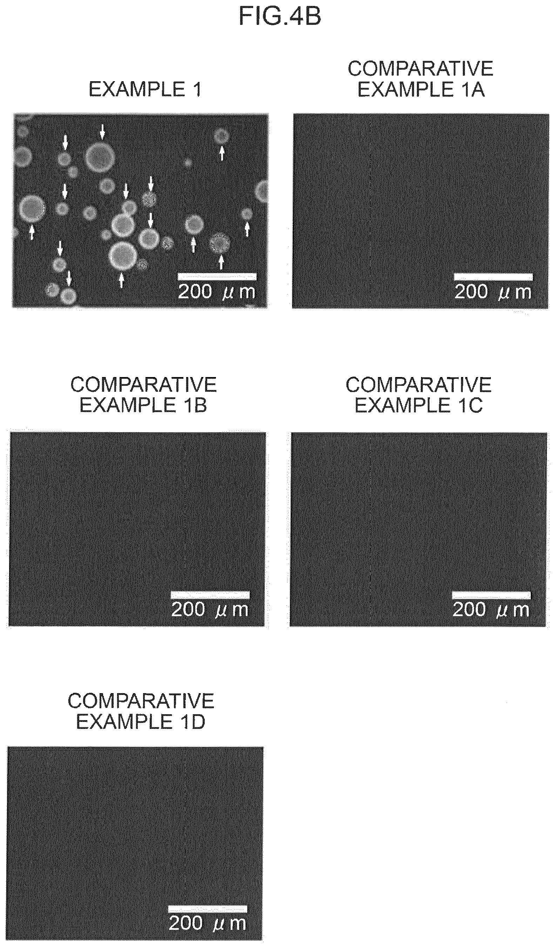

The results are illustrated in Figs. 4A and 4B. Figs. 4A and 4B are photographs in which the avidin-modified beads on the glass bottom dishes were measured. Fig. 4A illustrates phase-contrast images, and Fig. 4B illustrates fluorescence images. Each of the photos in Figs. 4A and 4B illustrates, from the left, the respective results of Example 1 and Comparative Examples 1A to 1D, and the arrows in these figures indicate those beads generating fluorescent signals. As illustrated in Fig. 4A, avidin-modified beads were observed in every photograph. Moreover, as illustrated in Figs. 4A and 4B, fluorescent signals were not observed in the regions where the avidin-modified beads were observed in the phase-contrast images for the Comparative Examples 1A to 1D. On the other hand, fluorescent signals were observed for Example 1 in the regions where the avidin-modified beads had been observed in the phase-contrast image, as indicated by the arrows.

Next, a microscope spectrometer (ECLIPSE TE300, manufactured by Nikon Corporation) was used to measure a spectrum of a fluorescent signal at 400 nm or above for a dispersion of Example 1 and a dispersion of Comparative Example 1B under excitation light at 340 nm ± 10 nm. The spectrum was also measured for a control configured similarly except in that the PBS was employed.

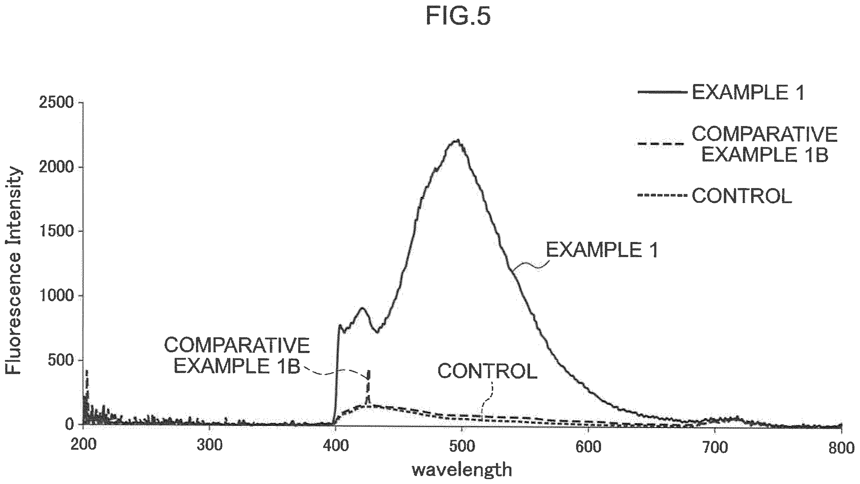

The results are illustrated in Fig. 5. Fig. 5 is a graph illustrating the spectra of fluorescent signals. In Fig. 5, the horizontal axis indicates wavelength, and the vertical axis indicates fluorescence intensity. As illustrated in Fig. 5, in the Comparative Example 1B, the fluorescent signal was about the same as in the control at 450 nm to 510 nm, this being where the fluorescent signal for pyrene appears. By contrast, in Example 1, a peak in fluorescent signal was clearly observed at a wave length a little less than 500 nm.

Example 2

Analyzing chips having detection sections of different depths were manufactured and beads of different diameters were confirmed to be recoverable in the detection sections.

(1) Manufacturing Flow Path Structural Body

An upper plate 10b of the analyzing chip 1 in Fig. 1 was produced by the method given below. Note that the size of each portion and the material of the analyzing chip are as given below.

Reaction Section 13: depth: 2 mm, diameter: 8 mm, circular shaped, with a circular through hole of 6 mm diameter. Detection Section 15: width: 0.15 mm, depth: 0.15 mm, length: 0.3 mm, rectangular shaped. Flow Path 14: length: 3.3 mm, width: 0.5 mm, sloped shaped so as to change in depth from 1 mm to 0.15 mm on progression from the upstream side toward the downstream side. Material: Manufactured from polydimethylsiloxane (PDMS)

First, a flow path mold made of aluminum was manufactured by machining to form a mold for manufacturing a flow path structural body of the structure. PDMS prepolymer solution was poured into the flow path mold, and placed in an oven and cured by heating for 1 hour at 70°C. After heat curing, the cured PDMS molded body was gently peeled from the flow path mold to obtain the flow path structural body. Note that the PDMS prepolymer solution was prepared as follows. A PDMS prepolymer and a curing agent (a principal component and a curing agent of Silpot (trade name, manufactured by Dow Corning Toray Co., Ltd.)) at a weight ratio of 10:1 were mixed until being homogenized, the mixed solution was then placed in a desiccator and depressurized so that bubbles generated during mixing was removed.

(2) Preparation of Reaction Reagent Solution

Rhodamine-labeled silica beads (manufactured by micromod Partikeltechnologie GmbH), mannitol, and bovine serum albumin were mixed with distilled water to 0.05 w/v%, 2 w/v%, and 1 w/v%, respectively, so as to prepare the reaction reagent solution. Note that four types of beads were employed, whose average diameters were 300 nm, 800 nm, 5 μm, and 10 μm, respectively.

(3) Immobilization of Reaction Reagent

Five μL of the reaction reagent solution was dripped onto the surface of a 0.17 mm thick borosilicate glass plate, and a 0.5 mm diameter stainless steel ball was further placed on the dripped reaction reagent solution. The glass plate was then incubated in a thermo-hygrostat for 24 hours at a temperature of 25°C and a humidity of 0%, and the stainless steel ball was immobilized on the glass plate as the reaction reagent solution dried.

(4) Analyzing Chip

A surface to be formed as the flow path (a surface onto which the glass plate is to be adhered) of the flow path structural body shown in (1) above was treated with oxygen plasma. The treated flow path structural body and the glass plate were adhered to each other so as to prepare the analyzing chip. The adhering of them was performed such that the position of a reaction section in the flow path structural body was made to face the position of the reaction reagent on the glass plate. Analyzing chips were similarly prepared, except in that the detection sections 15 had different depths of 40 $\mu$m, 70 $\mu$m, or 150 $\mu$m, respectively.

(5) Analysis

After dripping 50 $\mu$L of phosphate buffer solution into the reaction section 13 of the analyzing chips, the analyzing chips were fixed to the stage 23 of the centrifuge 2 illustrated in Fig. 2

The stainless steel ball in the reaction section 13 of the analyzing chips was rotated (at 200 rpm) by magnetic force to dissolve the reaction reagents for 10 minutes. The analyzing chips were then rotated at 4,000 rpm (rotation radius of 50 mm) for 60 seconds, and the beads in the reaction section 13 were moved to the detection section 15.

Using a fluorescence microscope, excitation light at a wavelength of from 510 nm to 560 nm was then irradiated onto the beads that accumulated at the leading end of the detection section 15 of the analyzing chips, and the average fluorescence intensity and absorbance at a wavelength of 490 nm or above was measured.

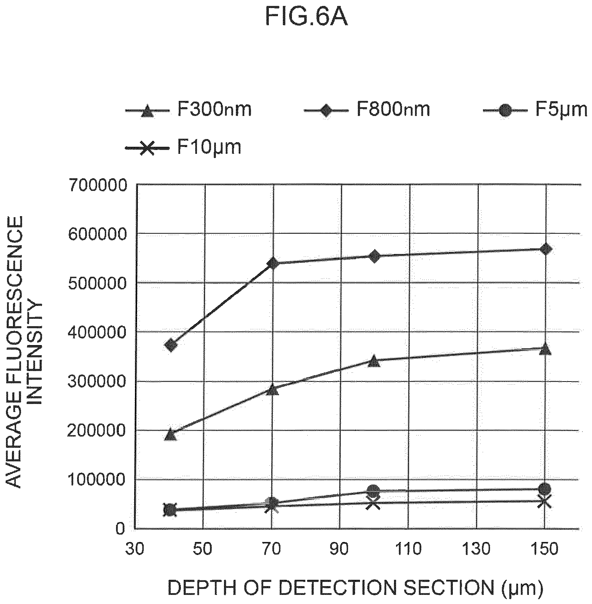

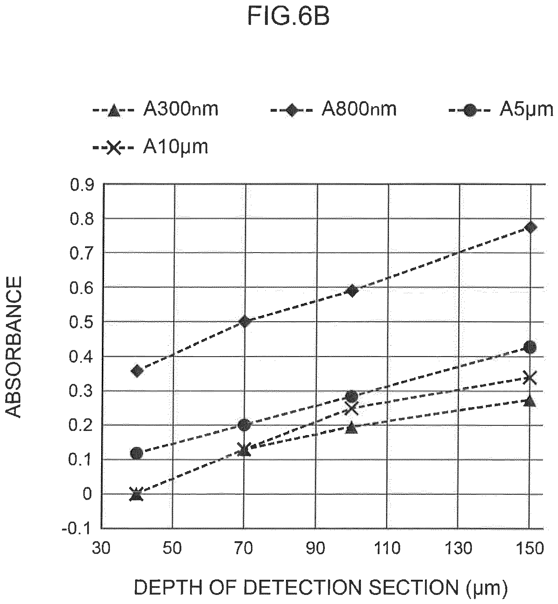

These results are illustrated in Figs. 6A and 6B. Figs. 6A and 6B are graphs illustrating average fluorescence intensity and absorbance. Fig. 6A illustrates the results for average fluorescence intensity, and Fig. 6B illustrates the results for absorbance. The triangles (△) in the figures indicate the results of the 300 nm-beads, the diamonds (◊) indicate the results of the 800 nm-beads, the circles (○) indicate the results of the 5 $\mu$m-beads, and the crosses (X) indicate the results of the 10 $\mu$m-beads. In Fig. 6A, the horizontal axis indicates the depth of the detection section 15, and the vertical axis indicates the average fluorescence intensity. In Fig. 6B, the horizontal axis indicates the depth of the detection section 15, and the vertical axis indicates the absorbance. As illustrated in Fig. 6A, increasing the depth of the detection section 15 increased the average fluorescence intensity for beads of every diameter. Among these beads, it was apparent that the 300 nm-beads showed high and constant rate of increase in average fluorescence intensity as the depth of the detection section 15 increased , and thus these beads found to be particularly appropriately employed as the carrier. As illustrated in Fig. 6B, as the depth of the detection section 15 increased, the absorbance for every bead diameter also increased. Moreover, it was apparent that the 300 nm-beads, the 5 $\mu$m-beads, and the 10 $\mu$m-beads showed reduced absorbance whatever depth was set for the depth the detection section 15, and thus, for example, detection misses might be reduced when detecting labeling using an optical method.

Example 3

It was confirmed that the beads were recoverable in the detection section employing the analyzing chips of varied radii of curvature of curved faces that constituted the side faces of the flow path 14 and that narrow in a tapered profile on progression from the reaction section 13 side toward the detection section 15 side and also employing surfactants of different concentrations.

(1) Analyzing chips

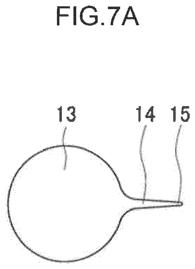

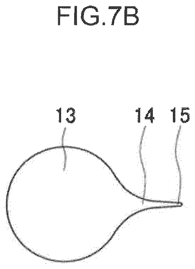

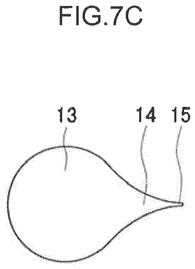

Analyzing chips were manufactured as shown in (1) to (4) of Example 2 above, except in that, as illustrated in Figs. 7A to 7C, the side faces of the flow paths 14 were configured with curved faces so as to narrow in a tapered profile on progression from the reaction section 13 toward the detection section 15 side, that the depth of the detection section 15 was set to 100 $\mu$m, and that rhodamine labeling silica beads were employed in the two types of beads of average diameter 300 $\mu$m and 5 $\mu$m. Note that in the analyzing chips illustrated in Figs. 7A, 7B and 7C, the radii of curvature R of the curved faces that narrow in a tapered profile were set to 1.5 mm, 3 mm, and 4.5 mm, respectively.

(2) Analysis

Fifty $\mu$L of a 10 mmol/L phosphate buffer solution (pH 7.4) containing 0.5 % Tween 20 (registered trademark) or Triton (trademark) X-100 was dripped into the reaction section 13 of the analyzing chips, and then the beads were accumulated in the detection section 15 as shown in (5) of Example 2 above. A fluorescence microscope was then used to irradiate excitation light of a wavelength from 510 nm to 560 nm onto the beads accumulated at the leading end of the detection section 15 of the analyzing chips, and the total sum of fluorescence intensities (total amount of fluorescence) of the accumulated portion of beads at a wavelength of 490 nm or above was calculated. The total amount of fluorescence before adding the beads was also calculated similarly, and the total amount of fluorescence was corrected by subtracting the calculated total amount of fluorescence before adding the beads from the calculated total amount of fluorescence after adding the beads (the total amount of fluorescence after correction).

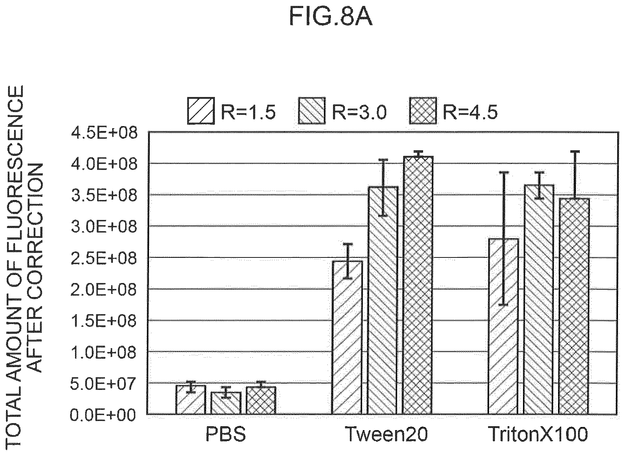

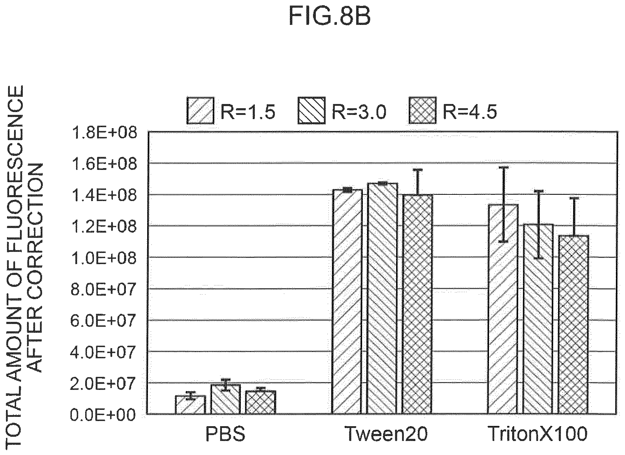

The results are illustrated in Figs. 8A and 8B. Figs. 8A and 8B are graphs illustrating the total amount of fluorescence after correction. Fig. 8A illustrates the results when the 5 µm-beads were used, and Fig. 8B illustrates the results of when the 300 µm-beads were used. In Figs. 8A and 8B, the horizontal axis indicates the type of surfactant, and the vertical axis indicates the total amount of fluorescence after correction. As illustrated in Figs. 8A and 8B, it is apparent that the total amount of fluorescence after correction was high and the beads were recoverable with good efficiency when the side faces of the flow path 14 were configured with curved faces of any of the radii of curvature. As illustrated in Figs. 8A and 8B, the total amount of fluorescence after correction was significantly increased in cases in which a surfactant (Tween 20 or Triton X-100) was included compared to cases in which a surfactant was not included (PBS). Namely, it is apparent that employing a surfactant as the reagent enabled the beads to be recovered with good efficiency.

Example 4

Three hundred µL of different serum samples A, B, and C including microRNA (miR-1246) as a target nucleic acid were each concentrated to 30 µL using a general genomic extraction kit and an RNA was extracted therefrom. Three µL measurement samples of each of the respective 30 µL extracted solutions were taken, and 27 µL of a reagent containing an appropriately adjusted pyrene-labeled probe, biotinylated probe, as well as an appropriate buffer solution and the like was added to be a total amount of 30 µL, which was quantitatively analyzed using a target analysis method as described in Example 1. In the quantitative analysis, an average value of three specimens for each of the serum samples was calculated from the values of the specimens obtained by matching the intensities of the observed fluorescent signals (see Fig. 5) against a detection amount curve generated in advance with known target nucleic acids.
The resulting target nucleic acid concentrations in the extracted solutions were 2.2 pmol/L, 2.5 pmol/L, and 1.3 pmol/L for the serum samples A, B, and C, respectively.
It was apparent from the results in Example 4 that target nucleic acid can be detected with high sensitivity even in very small amounts of samples by the target analysis method of the present embodiment.
From these results, it is apparent that a target in a sample can be directly analyzed by the analysis method of the present embodiment without using PCR.
Explanation is given above regarding the present embodiment with reference to exemplary embodiments. However, the present invention is not limited to the exemplary embodiments. It will be apparent to a person skilled in the art that various modifications to the configuration and particulars of the present embodiment can be made within the scope of the present invention.

Industrial Applicability

The present embodiment enables a target in a sample to be analyzed directly without using PCR. Moreover, in the first reaction process and the second reaction process of the analysis method of the present embodiment, the first bound body, including the target, the labeled probe, and the carrier-binding probe, can be concentrated on the carrier via the carrier-binding probe, and the carrier can be concentrated in the concentration process. Thus, in the analysis method of the present embodiment, the analyzing process, for example, enables a carrier in which the first bound body including the target is concentrated and bound (second bound body) to be analyzed in a highly concentrated state. Accordingly, the analysis method of the present embodiment, for example, enables excellent sensitivity and precision to be implemented, and enables analysis to be performed even, for example, when only a very small amount of the sample is provided. Moreover, the analysis method of the present embodiment, for example, enables the carrier to be analyzed in a highly concentrated state, thereby enabling a situation to be avoided in which the labeled probe not bound to the target affects the analysis. Therefore, the analysis method of the present embodiment reduces background noise, for example. Moreover, the analysis method of the present embodiment enables the binding reaction among the target, the labeled probe, and the carrier-binding probe to be performed in a dispersed state, and moreover enables the first bound body and the carrier to be bound together via the carrier-binding probe in the first bound body obtained. Thus, due to the higher probability of the binding reaction occurring between the labeled probe and the target dispersed in the reaction solution in the first reaction process, the analysis method of the present embodiment, for example, enables the time of the binding reaction to be further shortened and furthermore the sensitivity to be further improved, compared to analysis methods in which the carrier with immobilized labeled probe is employed to analyze the target, or analysis methods in which a bound body in which the carrier-binding probe and the carrier are preliminarily bound is employed to analyze the target. Accordingly, the present embodiment can be said useful in, for example, fields of treatment in which targets such as microRNA are analyzed.

Claims

1. A target analysis method comprising:

a first reaction process in which a target in a sample, a labeled probe that binds to the target, and a carrier-binding probe that binds to the target and to a carrier formed from a light-transmitting material contact each other in a liquid solvent, and the target, the labeled probe, and the carrier-binding probe react to bind to each other to form a first bound body;
a second reaction process in which the first bound body and the carrier contact each other, and the first bound body and the carrier react to bind to each other to form a second bound body having a higher specific gravity than that of the liquid solvent;
a concentration process in which the liquid solvent containing the second bound body is centrifuged to concentrate the second bound body; and
an analysis process in which the target in the sample is analyzed by detecting a label of the labeled probe contained in the concentrated second bound body.

2. The target analysis method of claim 1, wherein:

the carrier-binding probe includes a first binding substance;
the carrier includes a second binding substance to bind to the first binding substance; and
the carrier-binding probe binds to the carrier through a binding between the first binding substance and the second binding substance.

3. The target analysis method of claim 1 or claim 2, wherein the target is a target nucleic acid.

4. The target analysis method of claim 3, wherein the target nucleic acid is microRNA.

5. The target analysis method of claim 3 or claim 4, wherein the labeled probe is a fluorescent labeled probe.

6. The target analysis method of claim 5, wherein the fluorescent labeled probe binds closer to a 5' end side of the target nucleic acid than the carrier-binding probe.

7. The target analysis method of claim 6, wherein:

the fluorescent labeled probe binds to contiguous bases from the 5' end of the target nucleic acid; and
the carrier-binding probe binds to contiguous bases from a 3' end of the target nucleic acid.

8. The target analysis method of any one of claim 5 to claim 7, wherein:
a fluorescent label of the fluorescent labeled probe is a substance that undergoes a signal alteration through a binding of the fluorescent labeled probe to the target nucleic acid.

9. The target analysis method of claim 8, wherein the fluorescent label is a substance including pyrene.

10. The target analysis method of any one of claim 3 to claim 9, further including a process in which psoralen, which additionally modifies the labeled probe, and the target nucleic acid are caused to bind together by irradiation with ultraviolet light.

11. The target analysis method of any one of claim 1 to claim 10, wherein:

a target analyzing chip including a base plate is employed, the base plate being provided with a reaction section, a detection section, and a flow path that communicates the reaction section with the detection section;
the reaction section is a location where the target, the labeled probe, the carrier-binding probe, and the carrier react to bind to each other;
the detection section is a location where the second bound body is concentrated;
the flow path includes a hydrophobic internal wall as a movement control means to control movement of the second bound body from the reaction section to the detection section;
when a centrifugal force (C) greater than a resistance force (R) arising due to the hydrophobic properties of the flow path is applied, the second bound body can be moved by the movement control means to the detection

22

section through the flow path;

after a sample containing the target is introduced into the reaction section, the first reaction process is performed in the reaction section;

the second reaction process is then also performed in the reaction section;

the concentration process is performed en route from the reaction section to the detection section through the flow path; and

the analysis process is performed in the detection section.

12. The target analysis method of claim 11, wherein a height of the detection section is from 1 μm to 500 μm.

13. The target analysis method of claim 1, wherein the carrier is configured by a bead.

14. The target analysis method of claim 13, wherein the diameter of the bead is from 100 nm to 4 μm.

15. A target analyzing chip comprising:

a base plate provided with a reaction section, a detection section, and a flow path that communicates the reaction section with the detection section;

the reaction section being a location where a target in a sample, a labeled probe that binds to the target, and a carrier-binding probe that binds to the target and to a carrier formed from a light-transmitting material, and the carrier react to bind to each other;

the detection section being a location where a second bound body is concentrated, the second bound body being formed by a binding between a first bound body, in which the target, the labeled probe, and the carrier-binding probe are bound together, and the carrier via the carrier-binding probe;

the flow path including a hydrophobic internal wall as a movement control means to control movement of the second bound body from the reaction section to the detection section of; and

when a centrifugal force (C) greater than a resistance force (R) arising due to the hydrophobic properties of the flow path is applied, in the liquid solvent, the second bound body having a specific gravity higher than that of a liquid solvent can be moved by the movement control means to the detection section through the flow path.

## FIG.1A

## FIG.1B

FIG.1C

# FIG.2

FIG.3

## FIG.4A

EXAMPLE 1

COMPARATIVE
EXAMPLE 1A

COMPARATIVE
EXAMPLE 1B

COMPARATIVE
EXAMPLE 1C

COMPARATIVE
EXAMPLE 1D

# FIG.4B

EXAMPLE 1

COMPARATIVE
EXAMPLE 1A

COMPARATIVE
EXAMPLE 1B

COMPARATIVE
EXAMPLE 1C

COMPARATIVE
EXAMPLE 1D

FIG.5

EP 3 406 734 A1

# FIG.6A

# FIG.6B

FIG.7A

FIG.7B

FIG.7C

## FIG.8A

# FIG.8B

## INTERNATIONAL SEARCH REPORT

International application No.

PCT/JP2017/001280

### A. CLASSIFICATION OF SUBJECT MATTER

*C12Q1/68*(2006.01)i, *C12M1/00*(2006.01)i, *C12N15/09*(2006.01)i, *G01N21/78* (2006.01)i, *G01N35/00*(2006.01)i

According to International Patent Classification (IPC) or to both national classification and IPC

### B. FIELDS SEARCHED

Minimum documentation searched (classification system followed by classification symbols)
C12Q1/68, C12M1/00, C12N15/09, G01N21/78, G01N35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| Y/A | JP 8-062225 A  (Becton, Dickinson and Co.), 08 March 1996 (08.03.1996), entire text; particularly, claims & US 5639428 A claims & EP 693560 A2 | 1-14/15 |
| Y/A | WO 2002/097084 A1  (Hitachi, Ltd.), 05 December 2002 (05.12.2002), entire text; particularly, claims & US 2004/0116686 A1 claims & EP 1391507 A1 | 1-14/15 |

☒ Further documents are listed in the continuation of Box C.    ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 27 February 2017 (27.02.17) | 07 March 2017 (07.03.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

| International application No. |
| PCT/JP2017/001280 |

C (Continuation).   DOCUMENTS CONSIDERED TO BE RELEVANT

| Category* | Citation of document, with indication, where appropriate, of the relevant passages | Relevant to claim No. |
|---|---|---|
| Y/A | JP 2015-45579 A (Toray Industries, Inc.), 12 March 2015 (12.03.2015), entire text; particularly, claims; examples (Family: none) | 1-14/15 |
| Y/A | JP 2011-128019 A (Hitachi Maxell, Ltd.), 30 June 2011 (30.06.2011), entire text; particularly, paragraph [0057] (Family: none) | 1-14/15 |
| Y/A | WO 1999/51773 A1 (PHYLOS, INC.), 14 October 1999 (14.10.1999), page 14 & JP 2002-510505 A     & US 2002/0182597 A1 & EP 1068356 A | 1-14/15 |
| Y/A | US 2005/0014154 A1 (WEIZENEGGER Michael), 20 January 2005 (20.01.2005), paragraph [0037]; example 3 & JP 2005-507674 A     & WO 2003/039703 A2 & EP 1441825 A | 1-14/15 |
| A | JP 2003-524145 A (Gyros AB.), 12 August 2003 (12.08.2003), entire text; particularly, claims & US 2004/0202579 A1 claims & WO 1999/058245 A1     & EP 1077771 A | 15 |
| P,X | WO 2016/117700 A1 (Arkray, Inc.), 28 July 2016 (28.07.2016), entire text; particularly, claims; paragraph [0058] (Family: none) | 1-15 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5192229 B

- JP 4238381 B